# EUROPÄISCHE PATENTSCHRIFT

(11) **EP 0 977 894 B1**
(45) Veröffentlichungstag und Bekanntmachung des Hinweises auf die Patenterteilung: **25.06.2008**
(21) Anmeldenummer: 98931984.3
(22) Anmeldetag: 22.04.1998
(51) Int. Cl.: C12Q 1/68

(54) **VERFAHREN UND KIT ZUR IDENTIFIZIERUNG ANTIBIOTIKA-RESISTENTER BAKTERIENSTÄMME**
METHOD AND KIT FOR IDENTIFYING ANTIBIOTIC-RESISTANT STRAINS OF BACTERIA
PROCEDE ET KIT POUR IDENTIFIER DES SOUCHES BACTERIENNES RESISTANT AUX ANTIBIOTIQUES

(30) Priorität: 24.04.1997 DE 19717346
(43) Veröffentlichungstag der Anmeldung: 09.02.2000
(73) Patentinhaber: Max-Planck-Gesellschaft zur Förderung der Wissenschaften e.V., 80539 München (DE)
(72) Erfinder: HAKENBECK, Regine, D-12157 Berlin (DE)
(74) Vertreter: Schüssler, Andrea
(86) Internationale Anmeldenummer: PCT/DE1998/001134
(87) Internationale Veröffentlichungsnummer: WO 1998/048041

(56) Entgegenhaltungen:
- EP-A- 0 247 647
- WO-A-92/04458
- WO-A-96/08582
- LAIBLE G. ET AL.,: "Interspecies recombinational events during the evolution of altered PBP2x genes in penicliin-resistant clinical isolates of Streptococcus pneumoniae" MOL. MICROBIOL., Bd. 5, Nr. 8, - 1991 Seiten 1993-2002, XP002084616 in der Anmeldung erwähnt
- DOWSON C. G. ET AL.,: "Horizontal transfer of penicillin-binding protein genes in penicillin-resistant clinical isolates of Streptococcus pneumoniae" PROC. NATL. ACAD. SCI. USA, Bd. 86, - November 1989 Seiten 8842-8846, XP002084617

## Beschreibung

Die vorliegende Erfindung betrifft ein Verfahren und einen Kit zur Identifizierung Antibiotika-resistenter Bakterienstämme.

Das Auftreten Antibiotika-resistenter Bakterienstämme, insbesondere von Streptococcus-Stämmen, stellt ein wachsendes Problem dar. Bisher wurden Antibiotikasusceptibilitätstests so durchgeführt, daß Bakterien isoliert wurden, eine Kultur angelegt wurde, um die minimale Antibiotika-Hemmkonzentration in einem biologischen Test zu definieren. Dieses Verfahren nimmt mindestens 1 bis 2 Tage in Anspruch. Innnerhalb dieses Zeitraums kann nicht gezielt und damit nicht optimal therapiert werden. Es besteht deshalb ein Bedarf nach einer schnelleren Identifizierung bestehender Resistenzen.

Die WO 92/04458 beschreibt ein Verfahren und einen Kit zur Erkennung genetischer Elemente, die mit Antibiotikaresistenz assoziiert sind. Dazu wird eine isolierte Nukleinsäure mit verschiedenen Primern amplifiziert und mit DNA-Sonden, die spezifisch für Antibiotika-resistente Sequenzen sind, hybridisiert und detektiert. Unter anderem ist die Cefotaxim-Resistenz von Streptococcus pneumoniae durch mutiertes PBP2x beschrieben.

Die WO 96/08582 offenbart eine Methode zur Abschätzung bakterieller Resistenz gegen β-Lactam-Antibiotika. Dazu wird die bakterielle DNA mit einer Sonde in Kontakt gebracht, die spezifisch mit dem bakteriellen Antibiotika-Resistenzgen hybridisiert.

Die Aufgabe der vorliegenden Erfindung besteht darin, weitere Mittel und Verfahren bereitzustellen, mit denen Bakterienstämme, insbesondere Streptocokken-Stämme, schnell und zuverlässig auf bestehende Antibiotika-Resistenzen untersucht werden können.

Diese Aufgabe wird durch die in den Patentansprüchen angegebenen Gegenstände gelöst.

Die Erfindung wird nachfolgend anhand der Penicillin-Resistenz von Streptococcus pneumoniae beschrieben. Dieses Prinzip gilt jedoch auch entsprechend allgemein für Bakterien und für Resistenzen gegen andere Antibiotika. Beispielhaft seien Neisserien und MRSA-Stämme (Methicillin-resistente Staphylococcus aureus), die keine β-Lactamase produzieren, genannt.

Alle Penicillin-resistenten S. pneumoniae-Stämme besitzen veränderte Penicillin-Targetproteine (Penicillin-bindende Proteine, PBP). Die DNA-Sequenzen von Genen, die bei der Entwicklung von Penicillin-Resistenz in Streptococcus pneumoniae eine ausschlaggebende Rolle spielen, sind inzwischen in einer Anzahl von penicillinresistenten Streptokokken-Stämmen bestimmt worden. Es wurden drei Gene identifiziert, in denen bei der Entwicklung einer Penicillin-Resistenz zwischen sensitiven und resistenten Stämme Unterschiede auftreten: PBP2x, PBP1 a und PBP2b.

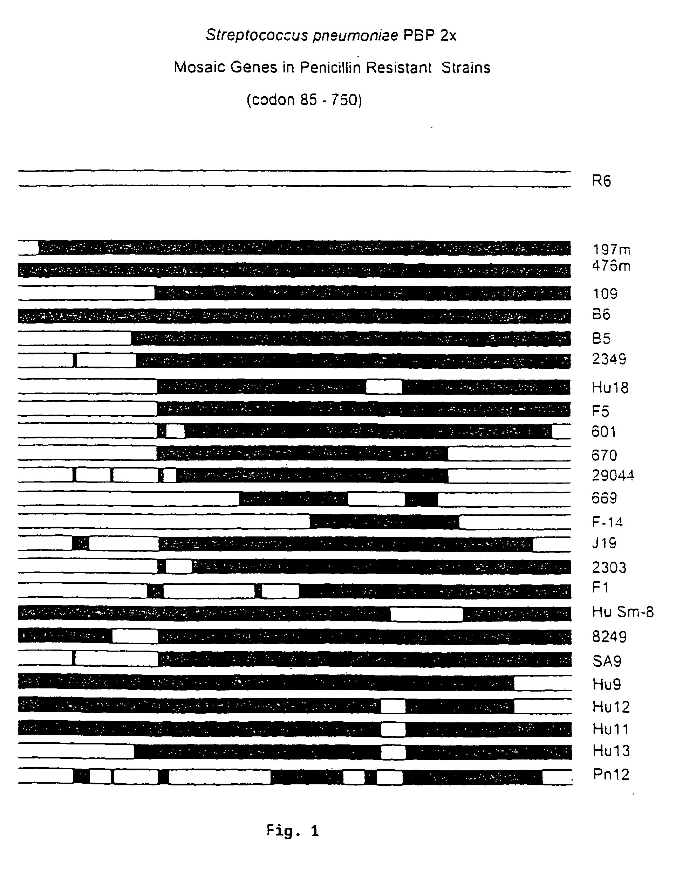

Ein Vergleich der DNA-Sequenzen zeigt innerhalb der Gene Bereiche auf, die in allen sensitiven S. pneumoniae Stämmen vorhanden sind, aber in resistenten Stämmen verändert sind. In diesem Zusammenhang wird auf Fig. 1 verwiesen, wo gezeigt ist, daß sich die resistenten Stämme vom sensitiven Stamm R6 im PBP2x Gen mehr oder weniger deutlich unterscheiden, aber auch untereinander Unterschiede aufweisen.

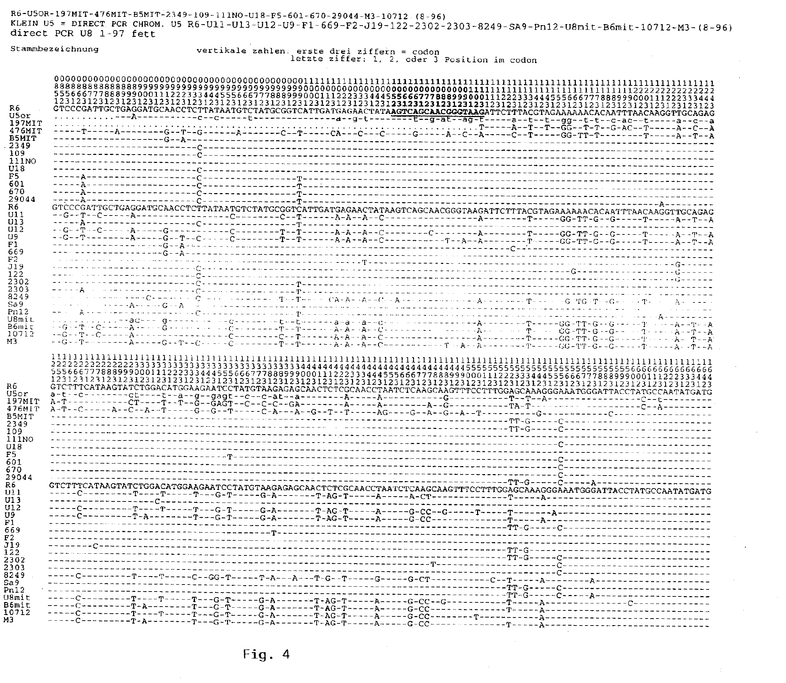

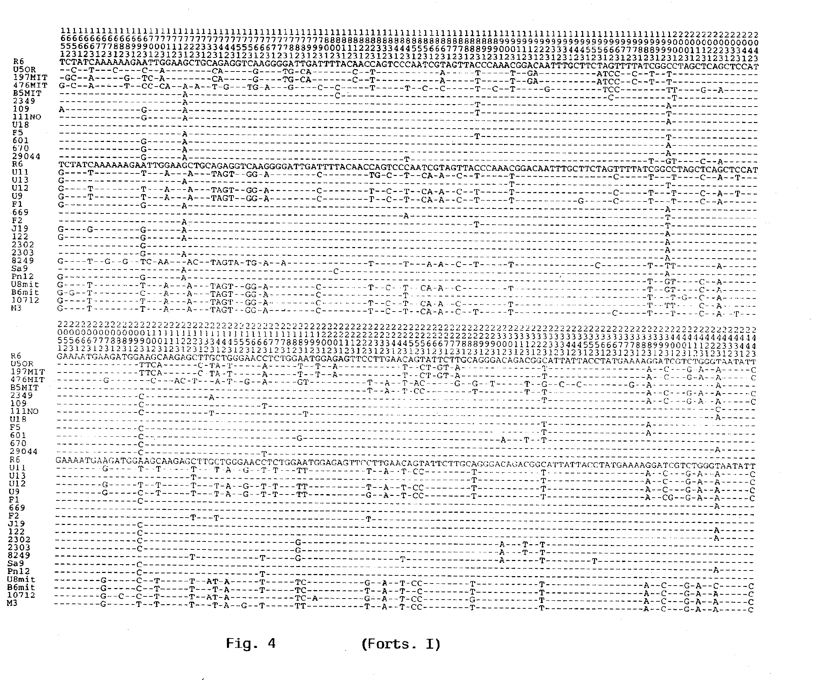

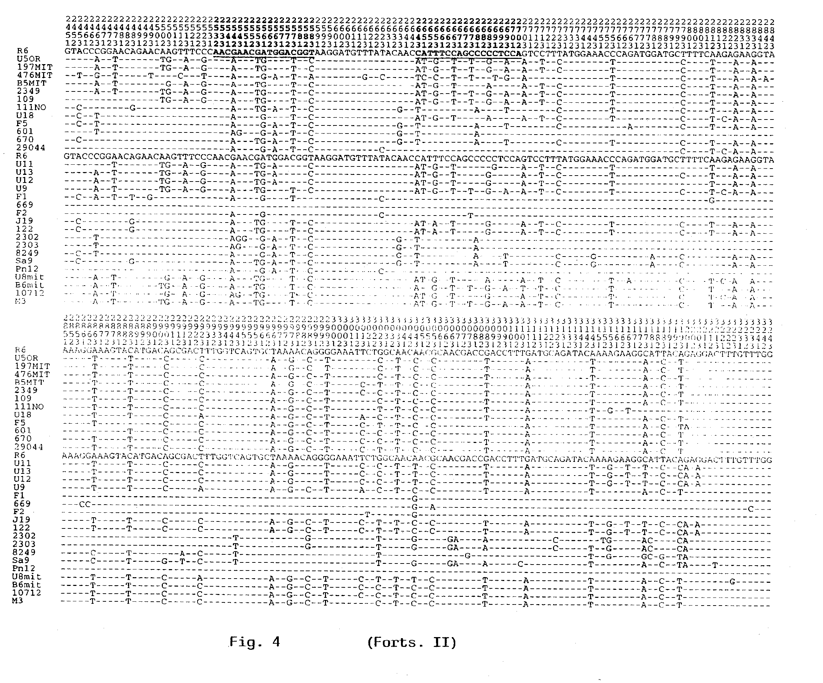

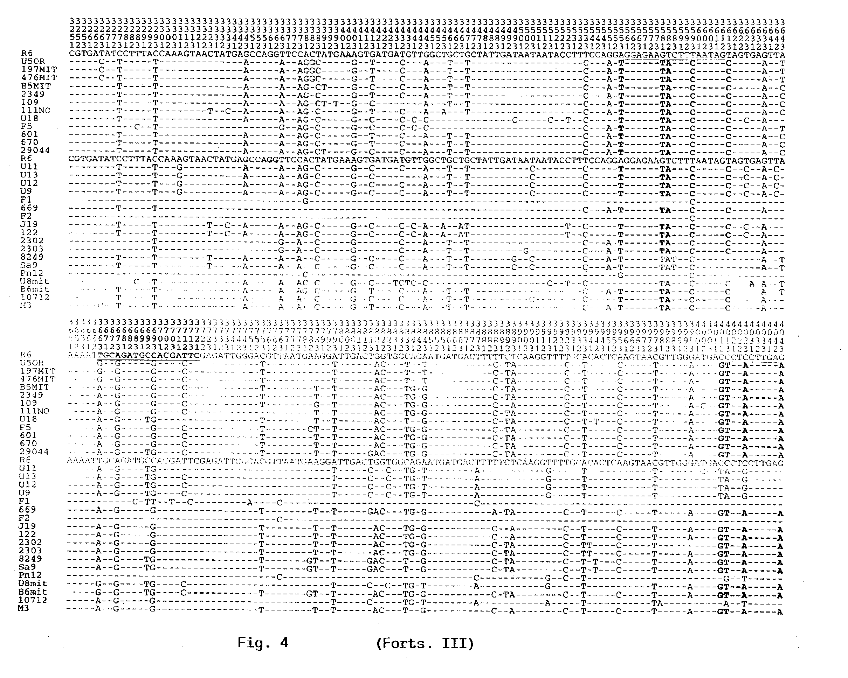

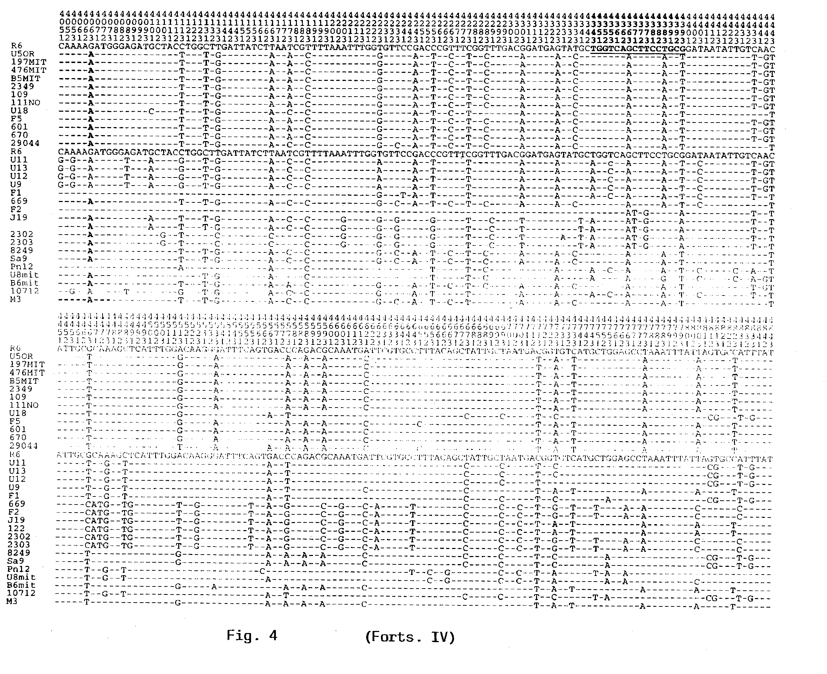

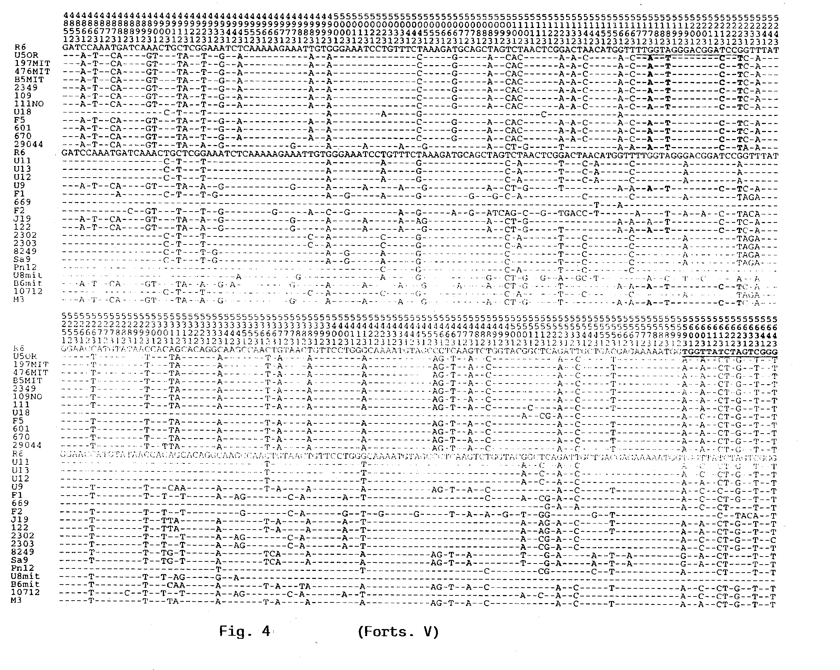

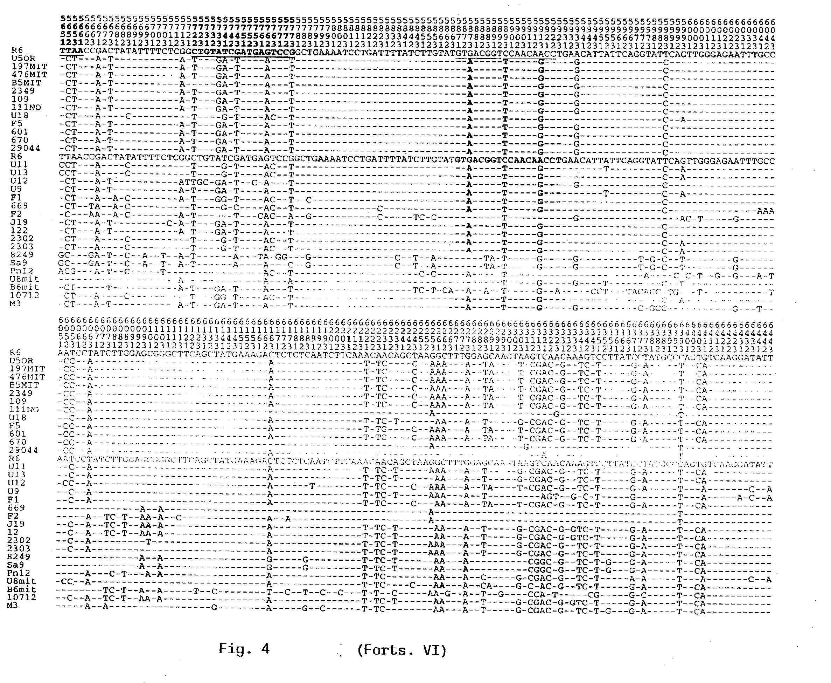

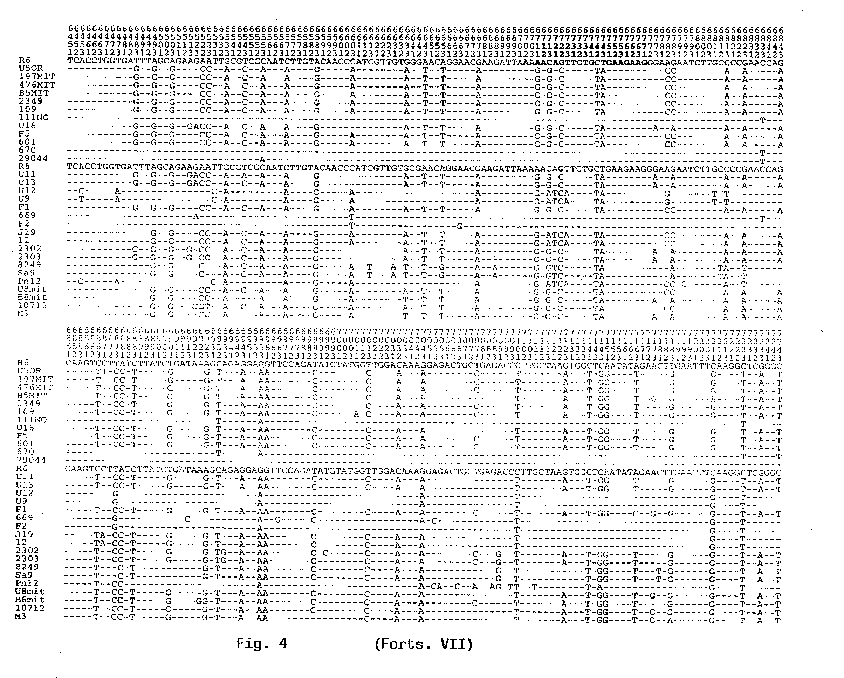

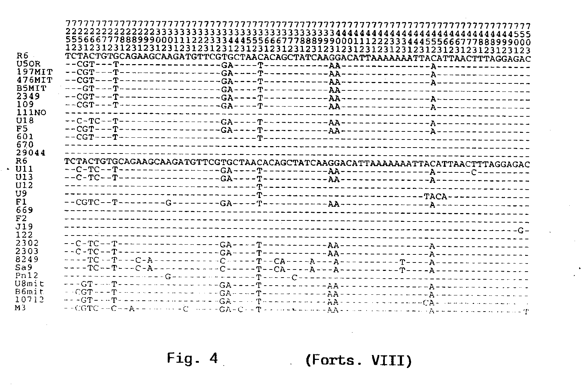

Aufgrund der obigen Erkenntnis, daß es innerhalb bestimmter Gene Unterschiede zwischen Penicillin-sensitiven und -resistenten Stämmen gibt, wurden von der Anmelderin DNA-Sonden entwickelt, mit denen resistente und sensitive Stämme differenziert werden können. In diesem Zusammenhang wird auf Fig. 4 verwiesen. Die Sonden, die spezifisch für sensitive Sequenzen sind, diskriminieren Gene, die für niedrigaffine PBP-Varianten codieren, welche für Penicillin-Resistenz verantwortlich sind. Die Sonden, die spezifisch für resistente Sequenzen sind, reagieren mit einer sehr häufig vorkommenden Klasse von PBP-Varianten und können auch für epidemiologische Zwecke verwendet werden.

Von der Anmelderin wurden folgende DNA-Sonden identifiziert:

| a) Sensitiv-spezifische Sonden für PBP2x. Die Zahlen unter der Rubrik "Nukleotid" beziehen sich auf die Nukleotide der publizierten Sequenz (Laible et al., Mol. Microbiol. 5, S. 1993-2002 (1991)). Die Zahlen in Klammern beziehen sich auf das Codon und die Position (1,2 oder 3) im Codon des Strukturgens. Die Anzahl der Basen im Nukleotid ist mit "mer" angegeben. | | |
|---|---|---|
| Nukleotid (Codon) | Oligonukleotid | -mer |
| 314-330 (105.2-110.3) | AGT CAG CAA CGG GTA AG (1) | 17 |
| 758-774 (253.2-258.3) | AAC GAA CGA TGG ACG GT (2) | 17 |
| 792-809 (264.3-270.2) | CAT TTC CAG NCC CCT CCA (3) | 18 (N:bevorzugt C) |
| 1098-1114 (366.3-372.1) | TGC AGA TGC CAC GAT TC (4) | 17 |
| 1302-1317 (434.2-439.3) | CTG GTC AGC TTC CTG CG (5) | 17 |
| 1677-1696 (559.3-566.1) | TGG TTA TCT AGT CGG GTT AA (6) | 20 |
| 1715-1731 (572.2-577.3) | CTG TAT CGA TGA GTC CG (7) | 17 |
| 2011-2029 (671.1-677.1) | AAC AGT TCT GCT GAA GAA G (8) | 19 |

| b) Resistenz-spezifische Sonden für PBP2x (wie oben; Sequenzen in Klam-mern entsprechen den korrespondierenden Abschnitten bei sensitiven Stämmen) | | |
|---|---|---|
| 1065-1084 (355.3-361.3) | (AGG AGA AGT CTT TAA TAG T) TGG AGA ATA NTT CAA TAG N (I) | 19 (N: bevorzugt C) |
| 1202-1221 (401.2-407.3) | (CCC TCC TTG AGC AAA AGA TG) GTC TAC TTG AAC AAA AAA TG (II) | 20 |
| 1549-1566 (517.1-522.3) | (TTG GTA GGG ACG GAT CCG) TTA GTT GGG ACG GAC CCT (III) | 18 |
| 1739-1776 (587.1-592.3) | (GTG ACG GTC CAA CAA CCT) GTA ACN NTT· CAA CAG CCT (IV) (GTG ACG GTC CAA CAA CCT) GTA ACN NTT CAA CAG CCT (IV) | 18 (N : bevorzugt G |

| c) Sensitiv-spezifische Sonden für PBP1a (Zahlen beziehen sich auf die Nukleotide der publizierten Sequenz des Strukturgens; Martin et al., EMBO J. 11, S. 3831-3836 (1992)) | | |
|---|---|---|
| (1034-1051) | TAG GAG CAC GCC ATC AGT (in den meisten bekannten Sequenzen spezifisch) | 18 |
| 1631-1648 | GAC GAA ATG CCT ATC TTG | 18 |
| 1722-1740 | CTC TCA ATT TGT AGC ACC T | 19 |
| 1794-1812 | CTA TTC TAA CCG TCT GAC A | 19 |

| d) Resistenz-spezifische Sonden für PBP1a | | |
|---|---|---|
| 945-963 | (TAC AGA CGA ATA CGT TGC C) CTC CGA NCA ATA CGT CTC T | 19 (N:bevorzugt T |
| 1735-1754 | (GCA CCT GAT GAA CTA TTT GC) GCT CCA GAT NAA ATG TTT GT | 20 (N:bevorzugt G |

| e) Sensitiv-spezifische Sonden für PBP2b (Zahlen beziehen sich auf die Nukleotide der publizierten Sequenz des Strukturgens; Hakenbeck, R., Matrin, C., Dowsen, C., Grebe, T., J. Bacteriol. 176, S. 5574-5577 (1996)) | | |
|---|---|---|
| 1329-1348 | ATC AAA TAC CTA TAT GGT CC | 20 |

| | | |
|---|---|---|
| N = beliebiges Nukleotid | | |

Die obigen bzw. durch ein oder mehrere Nukleotide, bevorzugt bis 4 Nukleotide, davon abweichende Sonden eignen sich bestens, um unbekannte Streptococcus pneumoniae Stämme auf Resistenzen gegen Penicillin zu untersuchen.

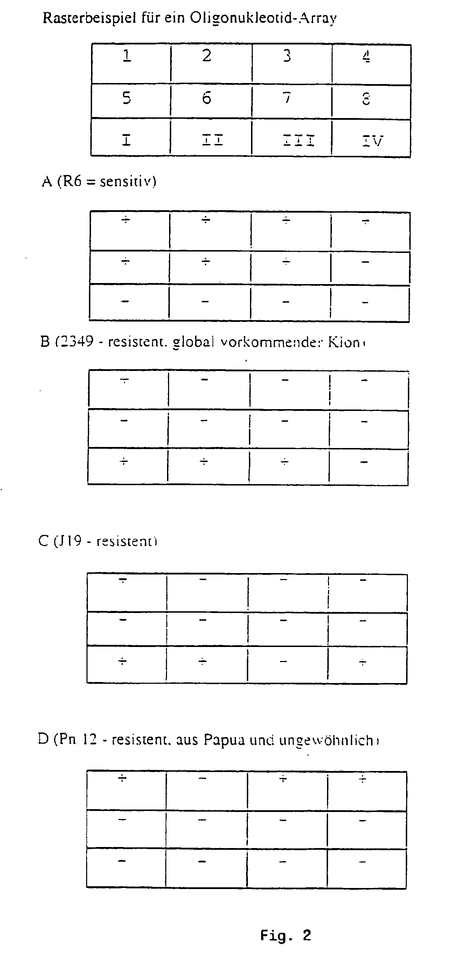

Dafür werden erfindungsgemäß Bakterien aus einer Probe abzentrifugiert und im Falle von S. pneumoniae die PBP-Gene (die Resistenzdeterminanten) direkt über PCR (polymerase chain reaction) wie in der Literatur beschrieben (Grebe und Hakenbeck (1996), Antimicrob. Agents Chemother. 40, S. 829-834) amplifiziert. Der Vorteil bei S. pneumoniae besteht darin, daß eine Detergenz-induzierte Lyse schnell erfolgt und damit eine PCR ohne langwierige DNA-Präparationen erfolgen kann. Da dieser Schritt bei anderen Streptokokken nicht gelingt, wird mit diesem Schritt spezifisch nur Pneumokokken-DNA amplifiziert. Alternativ wird bakterielle DNA (chromosomale und/oder extrachrosomale) gemäß Standardmethoden isoliert. Diese DNA wird mit mindestens einer Sensitiv-spezifischen Sonde und mit mindestens einer Resistenz-spezifischen Sonde unter Standardbedingungen, die dem Fachmann hinreichend bekannt sind, hybridisiert (s. z.B. Maniatis et al., Molecular Cloning, Cold Spring Harbor, N.Y.: Cold Spring Habor Laboratory). Bevorzugt erfolgt die Hybridisierung unter stringenten Bedingungen, wie z.B. 20°C unter dem Schmelzpunkt der hybridisierenden DNA. Die Oligonukleotide werden vorzugsweise so gewählt, daß sie ähnliche Schmelztemperaturen haben und somit mehrere in demselben Hybridisierungsansatz mit denselben Bedingungen getestet werden können (s. Fig. 2). Die Oligonukleotide werden bevorzugt markiert angeboten (P³², S³⁵, Biotin/Avidin-System; Dioxygenin (DIG)-markiert; Fluorescein-markiert) und gegen immobilisierte DNA hybridisiert. Alternativ werden die Oligonukleotide nicht markiert auf einem Oligonukleotid-Mikroarray angeboten, und die zu hybridisierende DNA über PCR gewonnen und bei der Amplifizierung markiert.

Aus dem Hybridisierungsergebnis kann man dann schließen, ob der unbekannte Stamm Antibiotika-sensitiv ist oder nicht. Bei der Hybridisierung sollte, je nach Resistenzgen, mindestens eine sensitiv-spezifische Sonde und eine resistenz-spezifische Sonde verwendet werden. Vorteilhafterweise wird die DNA des unbekannten Stamms jedoch mit mehreren sensitiv-spezifischen und Resistenz-spezifischen Sonden nacheinander hybridisiert, da eine Resistenz-Abschätzung mittels nur einer Kombination von sensitiv-spezifischen Sonden und Resistenz-spezifischen Sonden mit Fehlern behaftet sein kann und eher nur als grobe Abschätzung diesen kann. Dies gilt insbesondere für den Fall der Penicillinresistenzen bei Pneumokokken und Neisserien.

Bevorzugte Hybridisierungsbedingungen richten sich nach dem AT-Gehalt und Länge der Oligonukleotide. Die Wahl der geeigneten Bedingungen kann der Fachmann mit seinem Fachwissen ausführen. So werden beispielsweise 10-100 ng/ml markiertes Oligonukleotid für PBP2x (s.oben) bei einer Hybridisierungstemperatur von 45°-60°C mindestens 5 Stunden, bevorzugt über Nacht, in SSC-Hybridisierungslösung eingesetzt.

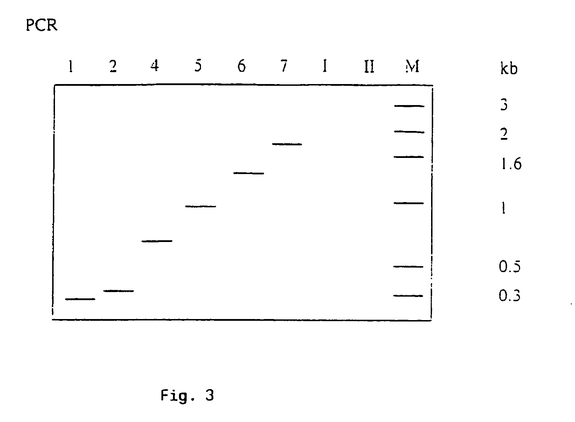

Die Oligonukleotide können auch als PCR-Primer verwendet werden, um damit einen PCR-Test aufzubauen (s. Fig. 3). Bei diesem Test kann auf die etwas zeitaufwendigere Hybridisierung verzichtet werden, allerdings müssen pro Stamm mehrere PCRs angewendet werden. Diese Methode ist vor allem für epidemiologische Zwecke geeignet.

Die Tatsache, daß weniger Sonden für PBP1a und besonders für PBP2b bekannt sind, ergibt sich aus der Tatsache, daß in PBP1a und besonders in PBP2b kleinere Genbereiche für die Resistenz von Bedeutung sind und daher auch nur kleinere Bereiche eine Sequenzvariation aufweisen.

Die Erfindung betrifft weiter einen Kit zur Durchführung des obigen Verfahrens. Dieser Kit umfaßt Mittel zur Isolierung von DNA aus Bakterien bzw. zur PCR-Amplifikation spezifischer Resistenzdeterminanten, Sensitiv-spezifische DNA-Sonden und Resistenz-spezifische DNA-Sonden (lyophilisiert bzw. als Oligonukleotid-Microarray), Reagenzien, Lösungen, Puffer sowie Mittel zur Hybridisierung und zum anschließenden Nachweis hybridisierter DNA. Die Sensitiv-spezifischen DNA-Sonden und Resistenz-spezifischen DNA-Sonden sind bevorzugt die oben aufgelisteten.

Die vorliegende Erfindung weist den Vorteil auf, daß innerhalb kürzester Zeit, d.h. innerhalb weniger Stunden, Bakterien, insbesondere Pneumokokken, hinsichtlich Antibiotika-Resistenz beurteilt werden können. Dies ermöglicht nachfolgend eine gezielte und effiziente Behandlung erkrankter Patienten.

Die Erfindung wird weiter anhand der Figuren beschrieben, welche zeigen:
- Fig.1: zeigt den Vergleich von Genabschnitten des Streptococcus pneumoniae PBP2x-Gen zwischen Penicillin-sensitiven und -resistenten Stämmen; Codon 85-750
R6: Penicillin-sensitiver Stamm
andere: Penicillin-resistente Stämme
- Fig. 2: zeigt die Hybridisierung auf einem Oligonukleotid-Array Die Anordnung der Sonden auf dem Array ist im ersten Block der Figur angegeben. Die Zahlen (1) bis (8) bzw. (I) bis (IV) entsprechen der Nummerierung der oben angegebenen Sonden für PBP2x.
A) Stamm R6, eine sensitiver S. pneumoniae Laborstamm und stellvertretend für andere sensitive Stämme: alle Sensitivspezifischen Oligonukleotide (Nr. 1-8) werden erkannt, während alle vier Resistenz-spezifischen Oligonukleotide (I-IV) nicht erkannt werden.
B) Stamm 2349, dessen PBP2x-Gen zu einer häufig und global vorkommmenden Klasse von PBP2x-Genen resistenter Pneumokokken gehört. Nur eines der Sensitiv-spezifischen Oligonukleotide wird erkannt, da die veränderte Sequenz nicht den 3'-Bereich des Gens überdeckt. Alle anderen Sensitivspezifischen Oligonukleotide (Nr. 2-8) hybridisieren nicht. Alle Resistenz-spezifischen Oligonukleotide (I-IV) hybridisieren.
C) Stamm J19, ein resistenter Stamm mit einem PBP2x, das nur teilweise Sequenzen hat, die mit dem von Stamm 2349 übereinstimmen. Eines der Resistenz-spezifischen Oligonukleotide (III) reagiert nicht.
D) Stamm Pn12, ein resistenter Stamm aus Papua, dessen PBP2x eine ungewöhnliche Sequenz aufweist. Fünf der Sensitiv-spezifischen Oligonukleotide reagieren nicht, ein Beweis, daß das PBP2x keine durchgehend sensitive Sequenz aufweist (und daher Resistenz vermittelt). Allerdings reagieren auch keine der Resistenz-spezifischen Oligonukleotide, was anzeigt, daß eine ungewöhnliche Sequenz auch in dem "resistenten" Genbereich vorliegt. Stämme wie dieser stellen die Ausnahme dar, können aber auf Grund des Screens eindeutig erfaßt werden, vor allem wenn weitere Oligonukleotide verwendet werden, die für andere PBPs spezifisch sind.
- Fig. 3: zeigt das Ergebnis von PCR-Reaktionen zur Amplifikation von S. pneumoniae R6-DNA als Auftrag auf einem Agarosegel. Als PCR-Primer wurden die oben mit (1) bis (7) gekennzeichneten PBP 2x-Sonden als "Forward-Primer" sowie jeweils Sonde (8) als "Reverse Primer" eingesetzt. Als Kontrolle wurden PCRs mit den Sonden (I) als forward-Primer und (IV) als reverse-Primer bzw. (II) als forward-Primer und (IV) als reverse-Primer durchgeführt. M = Größenmarker Auf dem gezeigten Gel ist klar zu erkennen, daß nur die Sensitivspezifischen Sonden eine Amplifikation ergeben, während keine mit Resistenz-spezifischen Sonden stattfindet.
- Fig. 4 (a) - (i): Ermittlung der erfindungsgemäßen Sonden durch Sequenzvergleiche

Die Erfindung wird weiter anhand eines Beispiels beschrieben.

### BEISPIEL: Isolierung von S. pneumomiae Bakterien-DNA und nachfolgende Testung auf bestehende Resistenz gegenüber Penicillin

Bakterien vom Stamm S. pneumoniae R6 werden in Brain-Heart-Infusion (BHI)-Medium überimpft und über Nacht bei 37°C wachsen gelassen. Die Zellen werden abzentrifugiert und durch Resuspendieren des Sediments in 10 µl 10 mM Tris/HCl-Puffer, pH 7,2, 0,05% Triton-X100 lysiert. Je 1 µl der Zellsuspension werden pro 20 µl PCR-Ansatz (0,2 µl Taq-Polymerase, je 1 pM Oligonukleotidprimer, 2 µl 10X PCR-Puffer, 4-6 mM MgCl₂) verwendet. Für die PCR-Reaktion sind 25 Zyklen mit 5 sec. Annealing 96°C, 5 sec. Annealing 52°C, 10 sec. Extension bei 72°C ausreichend.

### A) Agarose-Gelelektrophorese

In den PCR-Reaktionen (Bedingungen s. oben) werden folgende Primerkombinationen verwendet:

| forward-Primer | reverse-Primer |
|---|---|
| Sonde (1) | Sonde (8) |
| Sonde (2) | Sonde (8) |
| Sonde (3) | Sonde (8) |
| Sonde (4) | Sonde (8) |
| Sonde (5) | Sonde (8) |
| Sonde (6) | Sonde (8) |
| Sonde (7) | Sonde (8) |
| Sonde (I) | Sonde (IV) |
| Sonde (II) | Sonde (IV) |

Die Bezeichnungen der Sonden entsprechen den oben bei den Sequenzen angegebenen Zahlen für PBP2x.

Je 4 µl Aliquots der PCR-Reaktionen wurden auf ein 1,5%-iges AgaroseGel aufgetragen und elektrophoretisch aufgetrennt. Das Ergebnis ist in Fig. 3 gezeigt. Daraus ergibt sich, daß R6 ein sensitiver Stamm ist.

### B1) Dot-Blot

S. pneumoniae R6-Bakterien-DNA wird mit üblichen Primern (Grebe u. Hakenbeck (1996), Antimicrob. Agents Chemotherap. 40, S. 829-834) in einer PCR-Reaktion (Bedingungen s. oben) amplifiziert. Die PCR-amplifizierte DNA wird durch Erhitzen denaturiert (2 min. 96°C, anschließend 4°C), jeweils 2 µl davon werden pro Probe auf eine Nylon-Membran aufgetragen. Die DNA wird durch Bestrahlung mit langwelligem UV-Licht auf der Membran fixiert, unspezifische Bindungsstellen bei 60°C in Prähybridisierungslösung (6x SSC, 5x Denhardts-Lösung, 0,1 % SDS, 50 mM Na-Phosphat-Puffer pH 6,5, 0,1 mg/ml Heringsperm-DNA) unter leichtem Schütteln für 5 Std. abgesättigt. Die Hybridisierung mit den PBP2x Sensitiv-spezifischen Oligonukleotid-Sonden (1) bis (8) bzw. den Resistenz-spezifischen Sonden (I) bis (IV) erfolgt in Hybridisierungspuffer (wie Prähybridisierungslösung, jedoch mit 50 ng/ml Oligonukleotid-Sonde) über Nacht bei ca. 50°C. Das Filter wird 2 x 5 min. bei Raumtemperatur mit 2x SSC/0,1% SDS bei 55°C gewaschen. Die Proben werden mit anti-DIG-AP-Konjugat nach den Herstellerangaben (Boehringer Mannheim) angefärbt. Auch hier zeigt sich, daß nur die Sensitiv-spezifischen Sonden eine Hybridisierung ergeben, was anzeigt, daß der S. pneumoniae-Stamm R6 ein Penicillin-sensitiver Stamm ist.

### B2) Oligonukleotid-Mikroarray

Methodisch wird wie oben unter B1) vorgegangen, jedoch mit dem Unterschied, daß die Oligonukleotide als fertiger Array angeboten werden und die zu hybridisierende DNA über PCR mittels DIG oder fluoreszeinmarkierter Nukleotide markiert werden muß. Das Prinzip der "high density" Mikroarray-Hybridisierung ist in "Nature Biotechnology 14, S. 1675-1680, 1996" beschrieben. Das Ergebnis dieses Versuchs ist in Fig. 2 A gezeigt.

## Patentansprüche

1. Verfahren zur Identifizierung einer Antibiotikaresistenz bei Bakterien, wobei das Verfahren die folgenden Schritte aufweist:
(a) Isolierung von Bakterien-DNA
(b) Hybridisierung der in Schritt (a) gewonnenen DNA mit mindestens einer DNA-Sonde spezifisch für Antibiotika-sensitive Sequenzen und mindestens einer DNA-Sonde spezifisch für Antibiotika-resistente Sequenzen.

2. Verfahren nach Anspruch 1, wobei die Bakterien Streptococcus pneumoniae sind.

3. Verfahren nach Anspruch 1 oder 2, wobei die Antibiotikaresistenz eine Penicillinresistenz ist.

4. Verfahren nach einem der Ansprüche 1 bis 3, wobei die Sonden spezifisch für Antibiotika-sensitive Sequenzen ausgewählt sind aus den folgenden Oligonukleotiden bzw. sich davon durch ein oder mehrere Nukleotide unterscheiden und spezifisch für Antibiotika-sensitive Sequenzen sind:

AGT CAG CAA CGG GTA AG
AAC GAA CGA TGG ACG GT
CAT TTC CAG NCC CCT CCA
TGC AGA TGC CAC GAT TC

CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T
CTA TTC TAA CCG TCT GAC A
ATC AAA TAC CTA TAT GGT CC

5. Verfahren nach einem der Ansprüche 1 bis 4, wobei die Sonden spezifisch für Antibiotika-resistente Sequenzen ausgewählt sind aus den folgenden Oligonukleotiden bzw. sich davon durch ein oder mehrere Nukleotide unterscheiden und spezifisch für Antibiotika-resistente Sequenzen sind:

TGG AGA ATA NTT CAA TAG N
GTC TAC TTG AAC AAA AAA TG
TTA GTT GGG ACG GAC CCT
GTA ACN NTT CAA CAG CCT
CTC CGA NCA ATA CGT CTC T
GCT CCA GAT NAA ATG TTT GT.

6. Verfahren nach einem der Ansprüche 1 bis 5, wobei die Sonden radioaktiv markiert sind.

7. Kit zur Identifizierung Antibiotika-resistenter Bakterienstämme aufweisend Mittel zur Isolierung von DNA aus Bakterien bzw. zur PCR-Amplifikation spezifischer Resistenzdeterminanten, DNA-Sonden spezifisch für Antibiotika-sensitive Sequenzen und DNA-Sonden spezifisch für Antibiotika-resistents Sequenzen, Reagenzien, Lösungen, Puffer sowie Mittel zur Hybridisierung und zum anschließenden Nachweis hybridisierter DNA.

8. Kit nach Anspruch 7, wobei die Sonden spezifisch für Antibiotika-sensitive Sequenzen ausgewählt sind aus den folgenden Oligonukleotiden bzw. sich davon durch ein oder mehrere Nukleotide unterscheiden und spezifisch für Antibiotika-sensitive Sequenzen sind:

AGT CAG CAA CGG GTA AG
AAC GAA CGA TGG ACG GT
CAT TTC CAG NCC CCT CCA
TGC AGA TGC CAC GAT TC
CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T
CTA TTC TAA CCG TCT GAC A
ATC AAA TAC CTA TAT GGT CC

9. Kit nach Anspruch 7 oder 8, wobei die Sonden spezifisch für Antibiotika-resistente Sequenzen ausgewählt sind aus den folgenden oligonukleotiden bzw. sich davon durch ein oder mehrere Nukleotide unterscheiden und spezifisch für Antibiotika-resistente Sequenzen sind:

TGG AGA ATA NTT CAA TAG N
GTC TAC TTG AAC AAA AAA TG
TTA GTT GGG ACG GAC CCT
GTA ACN NTT CAA CAG CCT
CTC CGA NCA ATA CGT CTC T
GCT CCA GAT NAA ATG TTT GT

## Claims

1. A process for identifying an antibiotic resistance in bacteria, the process comprising the following steps:
(a) isolation of bacteria DNA,
(b) hybridization of the DNA obtained in step (a) with at least one DNA probe that is specific to antibiotic-sensitive sequences and at least one DNA probe that is specific to antibiotic-resistant sequences.

2. A process according to claim 1, wherein the bacteria are Streptococcus pneumoniae.

3. A process according to claim 1 or 2, wherein the antibiotic resistance is a penicillin resistance.

4. A process according to any of claims 1 to 3, wherein the probes that are specific to antibiotic-sensitive sequences are selected from the following oligonucleotides and/or differ therefrom by one or several nucleotides and are specific to antibiotic-sensitive sequences:

AGT CAG CAA CGG GTA AG
AAC GAA CGA TGG ACG GT
CAT TTC CAG NCC CCT CCA
TGC AGA TGC CAC GAT TC

CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T
CTA TTC TAA CCG TCT GAC A
ATC AAA TAC CTA TAT GGT CC

5. A process according to any of claims 1 to 4, wherein the probes that are specific to antibiotic-resistant sequences are selected from the following oligonucleotides and/or differ therefrom by one or several nucleotides and are specific to antibiotic-resistant sequences:

TGG AGA ATA NTT CAA TAG N
GTC TAC TTG AAC AAA AAA TG
TTA GTT GGG ACG GAC CCT
GTA ACN NTT CAA CAG CCT
CTC CGA NCA ATA CGT CTC T
GCT CCA GAT NAA ATG TTT GT.

6. A process according to any of claims 1 to 5, wherein the probes are radioactively labelled.

7. A kit for identifying antibiotic-resistant bacteria strains comprising agents for isolating DNA from bacteria and/or for the PCR amplification of specific resistance determinants, DNA probes that are specific to antibiotic-senstive sequences and DNA probes that are specific to antibiotic-resistant sequences, reagents, solutions, buffers and agents for hybridization and for the subsequent detection of hybridized DNA.

8. A kit according to claim 7, wherein the probes that are specific to antibiotic-sensitive sequences are selected from the following oligonucleotides and/or differ therefrom by one or several nucleotides and are specific to antibiotic-sensitive sequences:

AGT CAG CAA CGG GTA AG
AAC GAA CGA TGG ACG GT
CAT TTC CAG NCC CCT CCA
TGC AGA TGC CAC GAT TC
CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T
CTA TTC TAA CCG TCT GAC A
ATC AAA TAC CTA TAT GGT CC

9. A kit according to claim 7 or 8, wherein the probes that are specific to antibiotic-resistant sequences are selected from the following oligonucleotides and/or differ thereform by one or several nucleotides and are specific to antibiotic-resistant sequences:

TGG AGA ATA NTT CAA TAG N
GTC TAC TTG AAC AAA AAA TG
TTA GTT GGG ACG GAC CCT
GTA ACN NTT CAA CAG CCT
CTC CGA NCA ATA CGT CTC T
GCT CCA GAT NAA ATG TTT GT

## Revendications

1. Procédé pour identifier une résistance aux antibiotiques par les bactéries dans lequel le procédé comprend les étapes suivantes :
- (a) isolation de l'ADN de bactéries
- (b) hybridation de l'ADN recueilli à l'étape (a) avec au moins une sonde d'ADN spécifique pour les séquences sensibles aux antibiotiques et au moins une sonde d'ADN spécifique pour les séquences résistantes aux antibiotiques.

2. Procédé selon la revendication 1, **caractérisé en ce que** les bactéries sont Streptococcus pneumoniae.

3. Procédé selon la revendication 1 ou 2, **caractérisé en ce que** le résistant aux antibiotiques est un résistant à la pénicilline.

4. Procédé selon l'une des revendications 1 à 3, **caractérisé en ce que** les sondes sont choisies spécifiquement pour des séquences sensibles aux antibiotiques parmi les oligonucléotides suivants ou bien qui se différencient de ceux-ci par un ou plusieurs nucléotides et sont spécifiquement pour les séquences sensibles aux antibiotiques :

AGT CAG CAA CGG GTA AG
AAC GAA CGA TGG ACG GT
CAT TTC CAG NCC CCT CCA
TGC AGA TGC CAC GAT TC
CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T
CTA TTC TAA CCG TCT GAC A
ATC AAA TAC CTA TAT GGT CC.

5. Procédé selon l'une des revendications 1 à 4, **caractérisé en ce que** les sondes sont choisies spécifiquement pour les séquences résistantes aux antibiotiques parmi les oligonucléotides suivants ou bien qui se distinguent de ceux-ci par un ou plusieurs oligonucléotides et sont spécifiquement pour des séquences résistantes aux antibiotiques :

TGG AGA ATA NTT CAA TAG N
GTC TAC TTG AAC AAA AAA TG
TTA GTT GGG ACG GAC CCT
GTA ACN NTT CAA CAG CCT
CTC CGA NCA ATA CGT CTC T
GCT CCA GAT NAA ATG TTT GT.

6. Procédé selon l'une des revendications 1 à 5, **caractérisé en ce que** les sondes sont marquées de manière radioactive.

7. Kit d'identification d'un moyen incorporant des souches de bactéries résistantes aux antibiotiques pour l'isolation d'ADN à partir de bactéries ou pour l'amplification PCR de déterminants résistants spécifiques, de sondes ADN spécifiquement pour les séquences sensibles aux antibiotiques et des sondes ADN spécifiquement pour les séquences résistantes aux antibiotiques, des réactifs, des solutions, des solutions tampon, ainsi que des moyens pour l'hybridation et pour la preuve finale d'ADN hybrides.

8. Kit selon la revendication 7, **caractérisé en ce que** les sondes sont choisies spécifiquement pour les séquences sensibles aux antibiotiques parmi les oligonucléotides suivants ou bien qui se différencient de ceux-ci par un ou plusieurs nucléotides et sont spécifiquement pour les séquences sensibles aux antibiotiques :

AGT CAG CAA CGG GTA AG
AAC GAA CGA TGG ACG GT
CAT TTC CAG NCC CCT CCA
TGC AGA TGC CAC GAT TC
CTG GTC AGC TTC CTG CG
TGG TTA TCT AGT CGG GTT AA
CTG TAT CGA TGA GTC CG
AAC AGT TCT GCT GAA GAA G
TAG GAG CAC GCC ATC AGT
GAC GAA ATG CCT ATC TTG
CTC TCA ATT TGT AGC ACC T

CTA TTC TAA CCG TCT GAC A
ATC AAA TAC CTA TAT GGT CC.

9. Kit selon la revendication 7 ou 8, **caractérisé en ce que** les sondes sont choisies spécifiquement pour les séquences résistantes aux antibiotiques parmi les oligonucléotides suivants ou bien qui se différencient de ceux-ci par un ou plusieurs nucléotides et sont spécifiquement pour les séquences sensibles aux antibiotiques :
